# EUROPEAN PATENT SPECIFICATION

(11) **EP 4 110 237 B1**
(45) Date of publication and mention of the grant of the patent: **09.04.2025**
(21) Application number: 21761390.0
(22) Date of filing: 22.02.2021
(51) Int. Cl.: A61F 2/64, A61F 2/60, A61F 2/68, A61F 2/70, A61F 2/72, A61H 3/00, B25J 9/18, A61F 2/50, A61F 2/76

(54) **PROSTHETIC KNEE JOINT WITH ELECTRONICALLY-CONTROLLED TRANSMISSION**
KNIEGELENKPROTHESE MIT ELEKTRONISCH GESTEUERTEM GETRIEBE
ARTICULATION DE GENOU PROTHÉTIQUE À TRANSMISSION À COMMANDE ÉLECTRONIQUE

(30) Priority: 24.02.2020 US 202062980861 P
(43) Date of publication of application: 04.01.2023
(73) Proprietor: Vanderbilt University, Nashville, TN 37240 (US)
(72) Inventor: GOLDFARB, Michael, Nashville, Tennessee 37240 (US); CULVER, Steven C., Nashville, Tennessee 37240 (US)
(74) Representative: Algemeen Octrooi- en Merkenbureau B.V.
(86) International application number: PCT/US2021/019079
(87) International publication number: WO 2021/173495

(56) References cited:
- WO-A1-2018/217593
- US-A1- 2011 125 290
- US-A1- 2012 083 901
- US-A1- 2013 261 766
- US-A1- 2016 302 686
- US-A1- 2018 066 740
- US-A1- 2019 328 551

## Description

### FIELD OF THE INVENTION

This application is directed to a knee prosthesis or orthosis, and more specifically, to a knee prosthesis or orthosis that provides highly desirable transmission characteristics.

### BACKGROUND OF THE INVENTION

A typical gait cycle consists of a stance phase, during which the knee must support the weight of the body, and a swing phase, during which the knee allows the foot to swing forward in advance of the next stance phase. In the design of a knee prosthesis or orthosis, it is desirable for the knee joint to provide sufficient torque in the stance phase to support body weight. In swing phase, it is desirable for the knee to swing almost freely, and at much higher speeds than required during the stance phase. The knee should also provide torque in the swing phase, to support suitable knee motion, such appropriate knee flexion for clearance between the foot and ground, full knee extension at the end of swing, and damping to avoid high impact torques at full extension. Further, in the stance phase, it is desirable for the knee to support weight against knee flexion, but to freely allow knee extension, to allow straightening of the knee without the possibility of knee buckling. Further, in the knee extension portion of the swing phase, it is desirable to allow knee extension, while resisting knee flexion, so that the knee will support the user at heel strike immediately upon heel strike or foot strike. The knee must switch between these respective behaviors essentially instantaneously (e.g., within tens of milliseconds).

Recently, powered knee prostheses have emerged. These prostheses typically employ an electric motor that drives the knee joint through a high-gear transmission ratio (e.g., 200: 1). With a high-gear transmission ratio, the knee can provide the high torque required for stance phase, but is generally unable to provide low output impedance required for the swing phase of gait. Also, the unidirectional behavior must be achieved via sensing (of torque) and closed-loop control, which is less reliable and functional than mechanical means. It is possible to provide low impedance also with feedback control, but this also requires other sensing (of output torque), and is less reliable and functional than mechanical means. As such, implementing these behaviors via feedback control severely compromises performance, particularly relative to systems that provide these behaviors by design. The net effect is that these systems are unable to effectively provide the behavioral characteristics of unidirectionality, low-impedance, and high-speed, and thus are compromised in all three of the above-stated behaviors.

United States patent application US 2013/261766 A disclosed a powered prosthetic thigh that can have a proximal portion configured to couple to a prosthetic hip socket and that can have a distal portion attached to the proximal portion. The distal portion can have a distal connector configured to couple to a prosthetic knee. The powered prosthetic thigh can also have a computer controlled actuator configured to rotate the prosthetic thigh relative to the prosthetic hip socket.

United States patent application 2011/125290 A discloses a variable gain impedance controller for use in a control system for controlling a motorized prosthetic or orthotic apparatus provided with a joint. The controller comprises a sensor input for receiving a signal indicative of an interaction between the apparatus and the ground, a torque sensor input for receiving a signal indicative of the torque at the joint and a variable gain scheduler in communication with the sensor input so as to receive data therefrom thereby providing a variable torque gain. The variable gain impedance controller adjusts its control on the apparatus based on the variable torque gain and the indicated torque so as to a) increase the joint resistance to motion when the signal received from the sensor input indicates an interaction between the apparatus and the ground and b) decrease the joint resistance to motion when the signal received from the sensor input indicates an absence of interaction between the apparatus and the ground.

Some other approaches for knee prostheses with variable transmission ratios have been proposed, but these change ratios slowly, and therefore are unable to make substantial changes in gear ratio within a small fraction of a stride. These systems also do not provide unidirectional characteristics. In the absence of fast changes and unidirectional behaviors in the transmission ratio, these systems are unable to provide the above discussed behaviors within a stride and, therefore, cannot provide the desirable characteristics listed above.

It would be desirable to have a new prosthesis or orthosis that provides a more seamless, smoother, reliable and quicker transition between appropriate dedicated behaviors.

### SUMMARY OF THE INVENTION

The term embodiment and like terms are intended to refer broadly to all of the subject matter of this disclosure and the claims below. Statements containing these terms should be understood not to limit the subject matter described herein or to limit the meaning or scope of the claims below. Embodiments of the present disclosure covered herein are defined by the claims below, not this summary. This summary is a high-level overview of various aspects of the disclosure and introduces some of the concepts that are further described in the Detailed Description section below. This summary is not intended to identify key or essential features of the claimed subject matter. This summary is also not intended to be used in isolation to determine the scope of the claimed subject matter. The subject matter should be understood by reference to appropriate portions of the entire specification of this disclosure, any or all drawings and each claim.

According to the invention, a knee prosthesis or orthosis includes at least one moveable joint and a torque modulating unit. The torque modulating unit is configured to impose a controllable torque on the at least one moveable joint. The torque modulating unit includes at least one of a motor or brake. The at least one of the motor or brake coupled to the at least one moveable joint via an electronically-controlled transmission. The electronically-controlled transmission is a two-speed transmission.

### BRIEF DESCRIPTION OF THE DRAWINGS

The disclosure, and its advantages and drawings, will be better understood from the following description of exemplary embodiments together with reference to the accompanying drawings. These drawings depict only exemplary embodiments, and are therefore not to be considered as limitations on the scope of the various embodiments or claims.
FIG. 1 is a prosthesis or orthosis according to the invention.
FIG. 2A is a schematic of a high-gear transmission ratio (stance) for a two-stage planetary gear transmission according to the invention.
FIG. 2B is a schematic of a low-gear transmission ratio (swing) for a two-stage planetary gear transmission according to the invention.
FIG. 3A is a schematic of a high-gear transmission ratio (stance) for a three-stage planetary gear transmission according to the invention.
FIG. 3B is a schematic of a low-gear transmission ratio (swing) for a three-stage planetary gear transmission according to the invention.
FIG. 4 is a schematic chart depicting states of a ring clutch, a carrier clutch, and the resulting states of a knee prosthesis or orthosis according to the invention.
FIG. 5 is a torque modulating unit according to the invention.
FIG. 6 is a ring clutch according to the invention.
FIG. 7 is a ring clutch according to another embodiment.
FIG. 8 is a ring clutch according to a further embodiment.

While the invention is susceptible to various modifications and alternative forms, specific implementations have been shown by way of example in the drawings and will be described in further detail herein. It should be understood, however, that the invention is not intended to be limited to the particular forms disclosed. Rather, the invention may comprise modifications, equivalents, and alternatives falling within the scope of the invention as defined by the appended claims.

### DETAILED DESCRIPTION

According to the invention, a knee prosthesis or orthosis includes at least one moveable joint and a torque modulating unit. The torque modulating unit being configured to impose a controllable torque on the at least one moveable joint. The torque modulating unit includes at least one of a motor or brake. The at least one of the motor or brake being coupled to the at least one moveable joint via an electronically-controlled transmission. The electronically-controlled transmission being a two-speed transmission.

The knee prosthesis or orthosis of the present invention is advantageous in that it yields a knee joint that specifically provides: (1) high-torque, low-speed unidirectional extension torque, with little to no flexion torque (which is appropriate for the stance phase of the gait cycle); (2) low-torque, high-speed, low-impedance, bidirectional knee behavior (which is appropriate for the swing flexion phase of walking); and (3) low-torque, high-speed, low-impedance, unidirectional knee behavior in knee extension, with a simultaneous high resistance to knee flexion (which is appropriate for the swing extension phase of walking). The gear transmission ratios between the states are different by more than an order of magnitude, and the transmission is configured to change between these states under load on the order of less than 20 milliseconds, and desirably less than 15 or 10 milliseconds.

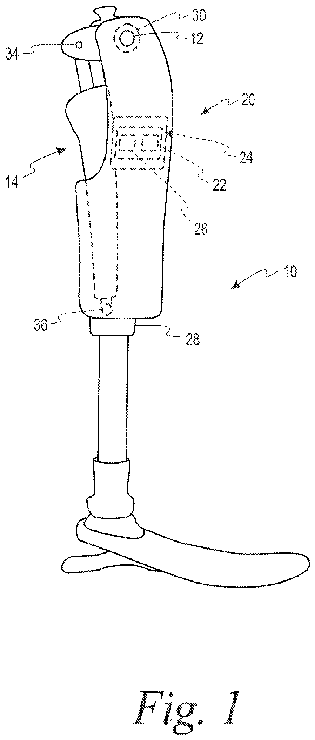

One non-limiting example of a knee prosthesis or orthosis that may be used is depicted in FIG. 1. The knee prosthesis or orthosis 10 includes at least one moveable joint 12 and a torque modulating unit 14. The knee prosthesis or orthosis 10 of FIG. 1 further includes a device control unit 20 that includes at least one accelerometer 22. The at least one accelerometer 22 may be located within an inertial measurement sensor (IMU) 24. The IMU 24 includes the at least one accelerometer 22 and at least one gyroscope 26. The IMU may be a multi-axis IMU in one embodiment. One non-limiting example of a multi-axis IMU includes a plurality of accelerometers and a plurality of gyroscopes. The knee prosthesis or orthosis 10 of FIG. 1 further includes a load cell 28 and a knee joint encoder 30. The load cell 28 assists in measuring the load of the knee prosthesis or orthosis 10. The knee joint encoder 30 assists in measuring knee joint angle.

The torque modulating unit 14 is configured to impose a controllable torque on the at least moveable joint 12. The knee prosthesis or orthosis 10 of FIG. 1 also further includes joints 34, 36 as shown in FIG. 1. The torque modulating unit 14 includes at least one motor or brake as will be discussed below. The at least one motor or brake of the torque modulating unit 14 is coupled to the at least one moveable joint 12 via an electronically-controlled transmission (ECT).

To provide a high-torque, low-speed, high-impedance behavior characteristic and also to separately provide a low-torque, high-speed, low-impedance behavior characteristic representative of knee behavior in stance and swing, respectively, the ECT used in the present invention is a two-speed transmission. Unlike common multi-speed transmissions, however, the two speeds in the ECT are spaced at least about an order of magnitude apart. For purposes of comparison, a standard automotive transmission typically changes gear ratios by a factor of 1.3 between adjacent gears. The gear transmission ratios used in the ECT of the present invention, however, should be at least about an order of magnitude different in ratio, in order to satisfy the torque, speed, and impedance differences associated with the stance and swing phases of gait. For example, in an embodiment, the gear transmission ratios in low gear and high gear should be at least a factor of 8 or at least a factor of 10 apart. In another embodiment, the transmission gear ratios should be different by a factor of 15. In a further embodiment, the transmission gear ratios should be different by a factor of 20.

In the embodiment described here, the two-speed transmission is based on a planetary gear transmission. The planetary gear transmission includes at least two transmission stages - an input planetary stage and an output planetary stage. In one embodiment, the input planetary stage includes at least an input sun gear, an input carrier link, and the output planetary stage includes an output carrier link. In this embodiment, the stages share a common ring gear (i.e., all stages share the same ring gear). Common ring gear means that the ring gears associated with each stage are rigidly coupled together.

In a typically embodiment, a sun gear, a carrier link, and at least three or more planet gears are used in each planetary stage (e.g., an input planetary stage and an output planetary stage). In this embodiment, each of the stages includes a common ring gear.

The two-speed transmission includes at least a ring clutch and a carrier clutch in one embodiment. The ring clutch and the carrier clutch are solenoid-actuated clutches in one embodiment. Both of the ring and the carrier clutches are configured independently to be either in an engaged state or a disengaged state. In the disengaged state, the ring clutch in one embodiment allows the common ring gear to rotate relative to ground. The term "ground" as used herein includes grounding to a frame, grounding to housing, or the like. In the engaged state, the ring clutch in one embodiment prevents or inhibits the common gear from rotating relative to ground. In the disengaged state, the carrier clutch in one embodiment allows the input carrier link to rotate relative to ground. In the engaged state, the carrier clutch in one embodiment prevents or inhibits the input carrier link from rotating relative to ground.

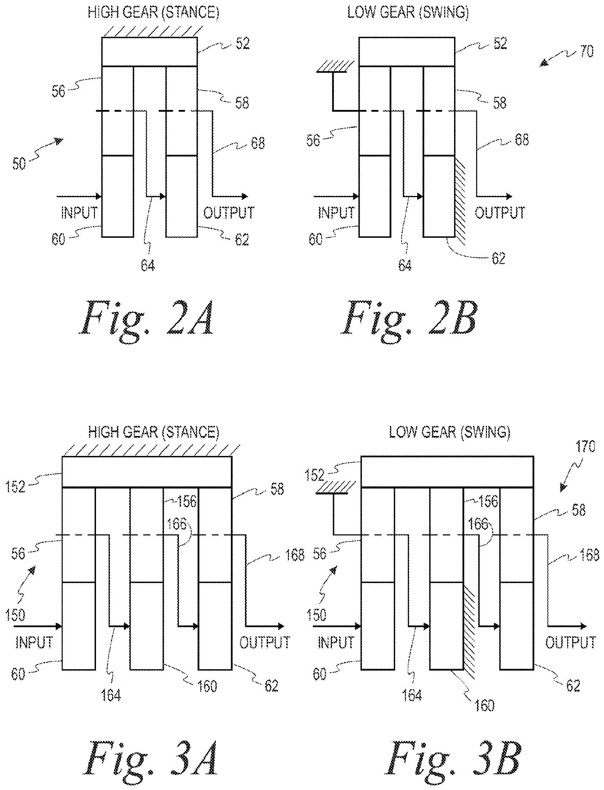

Referring to FIG. 2A, a schematic 50 of a high-gear transmission ratio (stance) for a two-stage planetary gear transmission according to the invention is shown. FIG. 2B depicts a schematic 70 of a low-gear transmission ratio (swing) for a two-stage planetary gear transmission according to the invention.

The schematic 50 of FIG. 2A includes a first stage (input) and a second stage (output) of a planetary gear transmission of a high-gear transmission ratio (stance). The schematic 70 of FIG. 2A, 2B includes a first stage (input) and a second stage (output) of a planetary gear transmission for a low-gear transmission ratio (swing). Thus, schematics 50, 70 of FIGS. 2A, 2B both depict exactly two stages.

Both of the schematics 50, 70 of the planetary gear transmission includes a common ring gear 52, an input planet gear 56, an output planet gear 58, an input sun gear 60, and an output sun gear 62. The input planet gear 56 is connected to the output sun gear 62 via an input carrier link 64. The output planet gear 58 is connected to an output carrier link 68. The input is the input sun gear 62 and the output is the last carrier link (carrier link 68 in schematics 50, 70).

In this embodiment, the input carrier link 64 can be grounded via a carrier clutch. In this embodiment, the carrier clutch is an electromagnetic clutch. In this embodiment, the common ring gear 52 is separately grounded by a ring clutch. The ring clutch in this embodiment is a unidirectional electromagnetic clutch. The carrier and ring clutches can be engaged or disengaged in various combinations to provide: (1) a high-gear transmission ratio appropriate for stance phase; (2) a low-gear transmission ratio appropriate for swing phase; and (3) a combination thereof that enables a low-gear transmission ratio against extension and high-impedance against flexion.

In the high gear (i.e., high-gear transmission ratio) of FIG. 2A, the input common ring gear 52 is locked to ground, while all of the other components remain free. In the low gear (i.e., low gear transmission ratio) of FIG. 2B, the common ring gear 52 is allowed to spin freely, while the input carrier link 64 and the output sun gear 62 are grounded. With this change of configuration between schematics 50, 70, differences in the gear transmission ratio of a factor of at least 15 to 20 are easily achievable.

In addition to a two-stage planetary gear transmission such as discussed above with respect to FIGS. 2A, 2B, a planetary gear transmission can have additional stages in other embodiments. For example, referring to FIGS. 3A, 3B, schematics of a three-stage planetary gear transmission are shown. More specifically, schematic 150 of FIG. 3A includes a first stage (input), a second stage, and a third stage (output) of a planetary gear transmission of a high-gear transmission ratio (stance). FIG. 3B depicts a schematic 170 that includes a first stage (input), a second stage and a third stage (output) of a planetary gear transmission of a low-gear transmission ratio (swing).

Both of the schematics 150, 170 of the planetary gear transmission includes an input common ring gear 152, the input planet gear 56, a second stage planet gear 156, the output planet gear 58, the input sun gear 60, a second stage sun gear 160, and the output sun gear 62. The input planet gear 56 is connected to the second stage sun gear 160 via an input carrier link 164. The second stage planet gear 156 is connected to the output sun gear 62 via an input carrier link 166. The output planet gear 58 is connected to an output carrier link 168.

In the high gear (i.e., high-gear transmission ratio) of FIG. 3A, the input common ring gear 52 is locked to ground, while all of the other components remain free. In the low gear (i.e., low gear transmission ratio) of FIG. 3B, the common ring gear 52 is allowed to spin freely, while the input carrier link 164 and the second stage sun gear 160 are grounded. With this change of configuration between schematics 150, 170, differences in the gear transmission ratio of a factor of at least 15 to 20 are easily achievable.

This general transmission configuration is configured to function for a planetary gear transmission with any number of stages provided that the ring gear of each stage is a common ring gear.

There are two defined gear transmission ratios (high-gear ratio and low-gear ratios.). In this embodiment there are two clutches (ring clutch and carrier clutch) on the transmission. In this embodiment, each of the ring and carrier clutches includes two states. The two states in the ring and carrier clutches are energized and de-energized. Because of the two gear transmission ratios and the two states, there are four possible states of transmission.

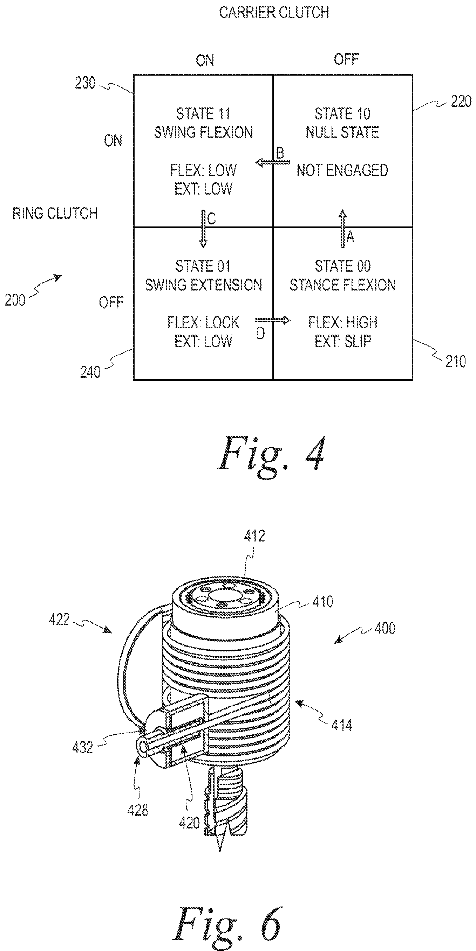

The sequence of clutch states that would be employed in a knee prosthesis or orthosis during walking are shown, for example, in FIG. 4. In FIG. 4, the axes represent an energized state of each ring clutch or carrier clutch. In the ON state, the ring and carrier clutches are energized, while in the OFF state, the ring and carrier clutches are de-energized. The ring and carrier clutches in this embodiment are electromagnetic. Referring to FIG. 4, a chart 200 is depicted with four states - a first state 210, a second state 220, a third state 230, and a fourth state 240. Arrows A-D of the chart 200 show the direction of the flow of the states during a gait cycle.

In each state, the configuration of the ring and carrier clutches produces one of four behaviors in each the flexion and extension directions: a low-gear transmission ratio (low), a high-gear transmission ratio (high), over-determined locking (lock), and under-determined motion between input and output (slip or not engaged). Each state of the transmission is described in more detail below.

In the first state 210, the ring clutch and the carrier clutch are both de-energized. The common ring gear in the first state 210 is grounded (only in one direction). The transmission is in its high gear against flexion. The high-gear transmission ratio is configured to support a weight of the prosthesis or orthosis of a user when standing on the knee prosthesis or orthosis. Because the ring clutch can only engage the transmission unidirectionally, the transmission slips against extension (i.e., does not resist extension movement). The unidirectional behavior of the transmission is configured to generate torque against knee flexion. The torque modulating unit is in the first state 210 when the prosthesis or orthosis user is standing on the prosthesis or orthosis (stance), providing support against flexion of the knee.

In the second state 220, the ring clutch is energized and the carrier clutch is de-energized. The transmission assumes its underdetermined state. Energizing the ring clutch unlocks the ring gear. With the ring gear unlocked, the transmission has an extra rotational degree of freedom, decoupling the output from the input. The motor can no longer provide torque at the knee joint. The torque modulating unit is only in the second state 220 for a few milliseconds before entering the third state 230. This is part of the transition from stance to swing, when the gear transmission ratio changes from high to low. The purpose of entering the second state 220 before entering the third state 230 is to avoid locking the transmission while it is moving, which has the potential to damage the transmission.

In the third state 230, the ring clutch and the carrier clutch are both energized. The ring gear is unlocked and free to rotate, and the first stage or input carrier link is locked to the housing, preventing or inhibiting its rotation. In this configuration, the transmission is in its low gear. The low-gear transmission ratio enables the prosthesis or orthosis to allow a knee joint to swing freely when the knee prosthesis or orthosis is not supporting the weight of the user. Because the carrier clutch provides bi-directional locking in this embodiment, the motor can provide torques in both directions at the knee. The torque modulating unit remains in the third state 230 until the prosthesis or orthosis has completed swing flexion and enters swing extension.

In the fourth state 240, the carrier clutch is energized and the ring clutch is de-energized. When the knee joint of the prosthesis or orthosis begins to extend while in swing phase, it is no longer necessary to energize the ring clutch. Because the ring clutch only provides unidirectional locking against flexion in this embodiment, and cannot resist extension, the ring clutch does not need to be energized during swing extension. The transmission is still in its low gear. If the prosthesis or orthosis is suddenly subject to a flexion load (e.g., if the prosthesis or orthosis user trips on something), then the prosthesis or orthosis automatically enters the high gear and is able to resist flexion. This mechanical fail-safe removes the lag-time from the controller that would otherwise have to switch the torque modulating unit into the high gear.

The low-gear transmission ratio is configured to have asymmetric behavior in which the two-speed transmission is configured to resist extension with a low resistance while resisting flexion with a high resistance.

In one embodiment, the two-speed transmission is configured to be in at least a first state, a second state or a third state. The two-speed transmission in the first state provides the high-gear transmission ratio when resisting flexion or providing extension, while providing little to no torque when resisting extension or providing flexion. The two-speed transmission in the second state provides the low-gear transmission ratio. The low-gear transmission ratio is bidirectional. The two-speed transmission in the third state provides the low-gear transmission ratio when resisting extension or providing flexion, while also providing a high resistance when resisting flexion or providing extension.

In another embodiment, the two-speed transmission is further configured for a fourth state. The two-speed transmission in the fourth state provides the high-gear transmission ratio. The high-gear transmission ratio is bidirectional.

In a further embodiment, the two-speed transmission is configured to be in at least a neutral state, in which the two-speed transmission is disengaged from at least one of the motor or brake.

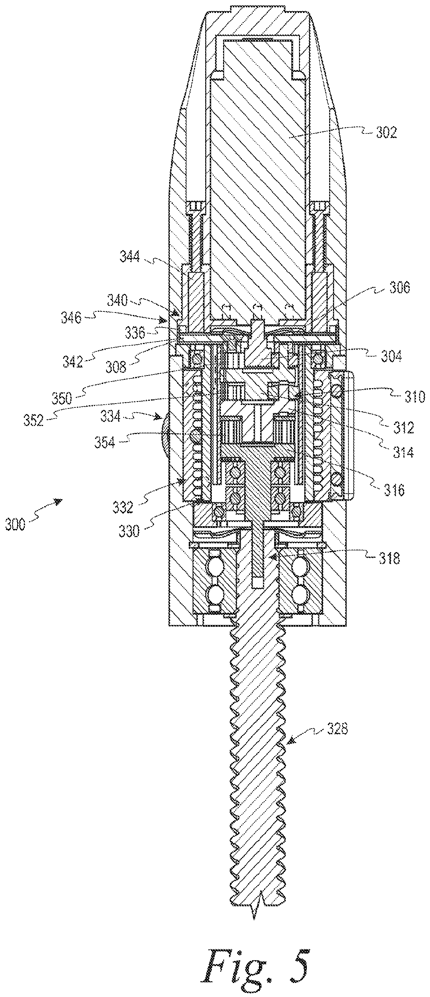

Referring to FIG. 5, a torque modulating unit 300 is shown according to the invention. The torque modulating unit 300 grounds the first stage carrier and the second stage sun-gear (which is rigidly connected to the first stage carrier). The torque modulating unit 300 includes an electric motor/brake 302, a first stage sun gear or motor output 304, a wave disk spring 306, a plain bearing 308, a planetary gear transmission 310 that includes a plurality of carriers links 312, 314, 316, a transmission output 318, a back-drivable lead screw 328, a common ring gear 330, a ring clutch capstan cable guide 332, a ring clutch solenoid 334, an O-ring 336, and a carrier clutch 340 that includes a carrier clutch armature 342 and a carrier clutch solenoid 344. The carrier clutch solenoid 344 and the plain bearing 308 form carrier clutch solenoid stator 346. The plain bearing 308 in one embodiment comprises ferrous steel.

The motor or brake 302 is coupled to at least one moveable joint (e.g., the at least one moveable join 12 in FIG. 1) via an electronically-controlled transmission. The motor or brake 302 is coupled to an input sun gear and at least one moveable joint being directly or indirectly coupled to the output carrier link. The electronically-controlled transmission is a two-speed transmission. The carrier link 312 is a first stage carrier link. The carrier link 312 is configured to translate along an input shaft. Upon energizing a solenoid affixed to ground, the magnetic field resulting from the solenoid pulls the carrier link 312 along the input shaft to make grounded contact. The carrier link 314 is a second stage carrier link, and the carrier link 316 is a third stage carrier link.

The torque modulating unit 300 is configured as an electromagnetic disk clutch. Thus, in this embodiment, the carrier clutch armature 342 is a ferromagnetic disk clutch armature. The carrier clutch armature 342 is collinearly fixed to the first stage carrier 312 of the planetary gear transmission 310. It is contemplated that the carrier clutch armature may be formed from materials other than ferromagnetic material.

The carrier clutch stator 346 is collinearly fixed to the electric motor/brake 302. The electric motor/brake 302 and the carrier clutch stator 346 are fixed to the housing. The planetary gear transmission 310 is supported by a pair of thin-section bearings so that it is able to freely rotate (when not constrained by the ring clutch or grounded). The wave disk spring 306 separates the carrier clutch armature 342 and the carrier clutch solenoid stator 346 when the torque modulating unit 300 is de-energized. The O-ring 336 is fixed to the carrier solenoid stator 346 and becomes compressed by the carrier clutch armature 342 when the carrier clutch 300 is energized. The plain bearing 308 encircles the carrier clutch armature 342 and the O-ring 336. The O-ring 336 in one embodiment is a medium durometer square-cross section O-ring.

When the clutch is de-energized, rotation and lateral translation of the carrier clutch armature 342 are constrained by the wave spring 306 and the plain bearing 308, respectively. When the ring clutch solenoid 334 is de-energized (i.e., in the OFF state), the spring element keeps the carrier clutch solenoid stator 346 and the carrier clutch armature 342 separated, and the O-ring 336 is not engaging the carrier clutch armature 342. When the ring clutch solenoid 334 is energized (i.e., in the ON state), the carrier clutch armature 342 is pulled to the carrier clutch solenoid stator 346 and is engaged by the O-ring 336, creating a high-friction interface between the stator 346 and the armature 342, locking the armature 342 to the stator 346, and preventing or inhibiting rotation of the armature 342. This prevents or inhibits rotation of the first stage carrier link and the second stage sun gear.

The ring and the carrier clutches desirably use friction-based locking that permits locking in any configuration. The ring and the carrier clutches desirably use solenoid-actuation permits state changes within several milliseconds, and therefore within a single stride of the gait cycle.

In one embodiment, the ring clutch is a unidirectional clutch. In the engaged state, the ring clutch allows unidirectional rotation of the common ring gear relative to ground. The unidirectional ring clutch is a capstan clutch in one embodiment.

In another embodiment, the ring clutch is selectively unidirectional or bidirectional. The ring clutch in this embodiment includes a first ring clutch and a second ring clutch. The engagement of the first and second ring clutches prevents or inhibits rotation of the common ring gear in both directions, and the selective disengagement of one of the first and second ring clutches allows unidirectional rotation of the common ring gear relative to ground in one of two directions. The selective disengagement of both the first and second ring clutches allows bidirectional rotation of the common ring gear relative to ground.

In a further embodiment, the ring clutch is a bidirectional clutch, such that in the engaged state, the ring clutch prevents or inhibits rotation of the common ring gear relative to ground in both directions.

Referring to FIG. 6, a ring clutch 400 is shown according to the invention. The ring clutch grounds the common ring gear, and is engaged during the stance phase to provide unidirectional holding against knee flexion. To provide a high holding torque, the ring clutch 400 employs a capstan-type design. It is contemplated that the ring clutch may not include a capstan-type design in other embodiments.

The design of the ring clutch 400 is beneficial in that it: (1) enables high holding torque with a low activation force, due to the exponential self-amplification property of a capstan; and (2) provides a unidirectional holding torque, which accommodates the previously described asymmetrical resistance desirable in the stance phase and late swing phase (i.e., low-impedance resistance to extension and high-impedance resistance to flexion).

The ring clutch of FIG. 6 includes a sleeve 410, a common ring gear 412, a wire rope 414, a spring element 420, an adjustable anchor point 422, a solenoid armature 428 and a solenoid stator 432.

The sleeve 410 is typically an aluminum sleeve. It is contemplated that the sleeve may be made of other materials, including other metallic materials. The sleeve 410 is pressed onto the common ring gear 412. The ring gear may be a ECT common ring gear. The wire rope 414 is wrapped a plurality of times around an exterior of the sleeve 410 as shown in FIG. 6. The wire rope 414 is typically a steel wire rope. It is contemplated that the wire rope may be made of other materials, including other metallic materials. The wire rope 414 is stiff and is compliant with the sleeve 410 and together form a capstan with a high static friction coefficient.

The spring element 420 applies tension to one end of the wire rope 414, while the other end of the spring element 420 is grounded to the housing by the adjustable anchor point 422. The adjustable anchor point 422 is adjustable via a set screw thereon that permits adjustment of the preload tension on the spring element 420. The distal ends of the spring element 420 and wire rope 414 are fixed to the solenoid armature 428. The solenoid armature 428 is located or positioned within the solenoid stator 432.

When the common ring gear 412 and the sleeve 410 are turned away from the end of the wire rope 414 that is secured to the adjustable anchor point 422, the force applied by the spring element 420 is multiplied by the capstan effect, producing a much larger holding force in the wire rope 414 secured to the anchor points. The effect is a strong holding torque that resists the motion of the rotating torque, effectively grounding the common ring gear. When a rotating torque is applied in the opposite direction (towards the ends of the wire rope 414 that is secured to the anchor point), the spring element 420 does not apply enough force to create a strong holding torque, and the drum rotates in the direction of the applied torque. This makes the ring clutch 400 a unidirectional clutch (i.e., the engagement that it provides is directionally dependent). The effect this has is similar to an overrunning clutch, where the rotating shaft inside the clutch is free to spin in one direction, but is locked in the opposite direction.

When the solenoid is de-energized (i.e., in the OFF state), the spring element 420 keeps the wire rope 414 engaged with the sleeve 410, and the common ring gear 412 can only be turned in one direction due to the capstan effect. When the solenoid is energized (i.e., in the ON state), the solenoid compresses the spring element 420, which negates the tensile force on the wire rope 414. The wire rope 414 is naturally stiff, and becomes straighter as force from the spring element 420 is removed. This causes the wire rope 414 to move away from the surface of the sleeve 410. With the wire rope 414 is no longer in contact with the drum, the clutch is disengaged, and the common ring gear 412 is free to rotate in both directions.

When the ring clutch solenoid is de-energized, the ring clutch is engaged. When the ring clutch solenoid is energized, the ring clutch is dis-engaged. When the carrier clutch solenoid is energized, the carrier clutch is engaged. When the carrier clutch is de-energized, the carrier clutch is disengaged.

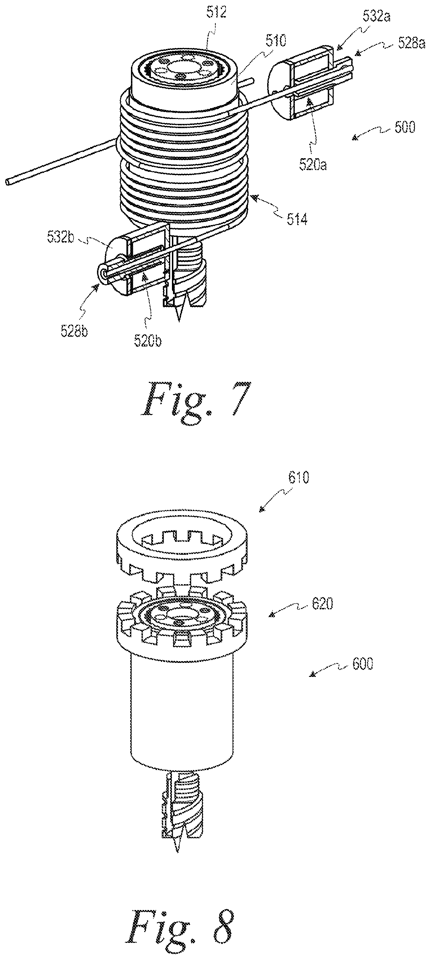

It is contemplated that other ring clutches may be used than the ring clutch shown in FIG. 6. Another non-limiting example of a ring clutch according to a further embodiment in shown in FIG. 7. The ring clutch 500 of FIG. 7 utilizes two solenoid-actuated unidirectional capstan clutches that are arranged in opposing orientations, such that using a second unidirectional locking clutch permits locking of the common ring gear in one of four configurations: (1) bidirectional locking, (2) locking to flexion only, (3) locking to extension only, and (4) bidirectional unlocking.

The ring clutch 500 of FIG. 7 includes a sleeve 510, a common ring gear 512, a wire rope 514, a plurality of spring elements 520a, 520b, a plurality of solenoid armatures 528a, 528b, and a plurality of solenoid stators 532a, 532b.

The flexion ring clutch and solenoid provides a grounding force for the common ring gear when a resistive knee flexion torque is applied, but permits knee extension with little or no resistance. The extension ring clutch and solenoid provides a grounding force for the common ring gear when a resistive knee extension torque is applied, but permits knee flexion with little or no resistance. The ends of each capstan for both clutches are grounded.

Referring to FIG. 8, a ring clutch 600 is depicted according to another embodiment. The ring clutch 600 utilizes a solenoid-actuated positive engagement clutch. The ring clutch 600 includes an upper portion 610 that is fixed to housing, and a lower portion 620 that is fixed to the common ring gear. The upper and lower portions 610, 620 of the ring clutch 600 have a geometry such that when the portions 610, 620 are pressed together by an actuating force (e.g., a solenoid), they interlock with one another. When the upper and lower portions 610, 620 are interlocked, an interface is created that prevents or inhibits the portions 610, 620 from rotating relative to one another (i.e., they form a dog clutch). The ring clutch 600 resists bidirectional rotation of the ring clutch. This embodiment can be combined with the ring clutch 400, such that the positive-engagement clutch shown here, which locks the ring gear in both rotation directions, can be used in parallel with a solenoid-actuated unidirectional-locking clutch, such that their combined use permits selective unidirectional or bidirectional locking of the ring gear.

The foregoing description of the embodiments, including illustrated embodiments, has been presented only for the purpose of illustration and description and is not intended to be exhaustive or limiting to the precise forms disclosed. Numerous modifications, adaptations, and uses thereof will be apparent to those skilled in the art.

Although the disclosed embodiments have been illustrated and described with respect to one or more implementations, equivalent alterations and modifications will occur or be known to others skilled in the art upon the reading and understanding of this specification and the annexed drawings. In addition, while a particular feature of the invention may have been disclosed with respect to only one of several implementations, such feature may be combined with one or more other features of the other implementations as may be desired and advantageous for any given or particular application.

While various embodiments of the present invention have been described above, it should be understood that they have been presented by way of example only, and not limitation. Numerous changes to the disclosed embodiments can be made within the scope of the invention as defined by the apended claims.

## Claims

1. A knee prosthesis or orthosis (10), comprising:
at least one moveable joint (12); and
a torque modulating unit (14, 300), the torque modulating unit (14, 300) configured to impose a controllable torque on the at least one moveable joint (12), the torque modulating unit (14, 300) including at least one of a motor or brake (302), the at least one of the motor or brake (302) coupled to the at least one moveable joint (12) via an electronically-controlled transmission, **characterized in that** the electronically-controlled transmission is a two-speed transmission.

2. The knee prosthesis or orthosis (10) of claim 1, wherein the two-speed transmission includes a high-gear transmission ratio and a low-gear transmission ratio, the two speed-transmission configured to switch between the high-gear transmission ratio and the low-gear transmission ratio during an intra-stride period during a gait cycle, the gait cycle including a stance phase and a swing phase,
wherein the high-gear transmission ratio provides a high-torque, low-speed, high-impedance behavior characteristic configured to the stance phase of the gait cycle, and wherein the high-gear transmission ratio is configured to support a weight of the prosthesis or orthosis (10) of a user when standing on the knee prosthesis or orthosis (10),
wherein the low-gear transmission ratio provides a high-speed, low-torque, low-impedance behavior characteristic suited to the swing phase of the gait cycle, and wherein the low-gear transmission ratio is configured to enable the prosthesis or orthosis (10) to allow a knee joint to swing freely when the knee prosthesis or orthosis (10) is not supporting the weight of the user.

3. The knee prosthesis or orthosis (10) of claim 2, wherein the high-gear and low-gear transmission gear ratios are different by at least about an order of magnitude.

4. The knee prosthesis or orthosis (10) of claim 2,
- wherein the high-gear transmission ratio is configured to have unidirectional behavior, wherein the two-speed transmission is configured to generate torque against knee flexion, but slips when resisting knee extension, and/or
- wherein the low-gear transmission ratio is configured to have asymmetric behavior, wherein the two-speed transmission is configured to resist extension with a low resistance while resisting flexion with a high resistance.

5. The knee prosthesis or orthosis (10) of claim 2, wherein the two-speed transmission is configured to be in at least a first state (210), a second state (220) or a third state (230), the two-speed transmission in the first state (210) provides the high-gear transmission ratio when resisting flexion or providing extension, while providing little to no torque when resisting extension or providing flexion; the two-speed transmission in the second state (220) provides the low-gear transmission ratio, the low-gear transmission ratio being bidirectional; and the two-speed transmission in the third state (230) provides the low-gear transmission ratio when resisting extension or providing flexion, while also providing a high resistance when resisting flexion or providing extension, wherein preferably the two-speed transmission is further configured for a fourth state, the two-speed transmission in the fourth state providing the high-gear transmission ratio, the high-gear transmission ratio being bidirectional.

6. The knee prosthesis or orthosis (10) of claim 2, wherein the two-speed transmission is configured to be in at least a neutral state, in which the two-speed transmission is disengaged from at least one of the motor or brake (302).

7. The knee prosthesis or orthosis (10) of claim 2, wherein the two-speed transmission includes a planetary gear transmission (310), the planetary transmission including at least an input planetary stage and an output planetary stage, the input planetary stage including at least an input sun gear (60) and an input carrier link (64, 164, 165), the output planetary stage including an output carrier link (68, 168),
wherein the at least one of a motor or brake (302) is coupled to the input sun gear (60) and at least one moveable joint (12) is directly or indirectly coupled to the output carrier link (68, 168),
wherein the planetary transmission further includes a common ring gear (52, 412, 512),
wherein the two-speed transmission includes at least a ring clutch (400, 500, 600) and a carrier clutch, the ring clutch (400, 500, 600) being configurable to be in an engaged state or a disengaged state, the carrier clutch being configurable to be in an engaged state or disengaged state,
wherein, in the disengaged state, the ring clutch (400, 500, 600) allows the common ring gear (52, 412, 512) to rotate relative to ground, and wherein in the engaged state, the ring clutch (400, 500, 600) prevents or inhibits the common gear from rotating relative to ground,
wherein, in the disengaged state, the carrier clutch allows the input carrier link (64, 164, 165) to rotate relative to ground, and wherein in the engaged state, the carrier clutch prevents or inhibits the input carrier link (64, 164, 165) from rotating relative to ground.

8. The knee prosthesis or orthosis (10) of claim 7, wherein the input planetary stage further includes at least a set of three input planet gears (56), and the output planetary stage further includes an output sun gear (62) and at least a set of three output planet gears (58).

9. The knee prosthesis or orthosis (10) of claim 7, wherein the ring clutch (400, 500, 600) and the carrier clutch are solenoid-actuated clutches.

10. The knee prosthesis or orthosis (10) of claim 8, wherein the input carrier link (64, 164, 165) is configured to translate along an input shaft, and wherein upon energizing a solenoid affixed to ground, the magnetic field resulting from the solenoid pulls the input carrier link (64, 164, 165) along the input shaft to make contact with ground.

11. The knee prosthesis or orthosis (10) of claim 9, wherein the ring clutch (400, 500, 600) is engaged when the ring clutch solenoid (334) is de-energized, and the ring clutch (400, 500, 600) is disengaged when the ring clutch solenoid (334) is energized; and wherein the carrier clutch is engaged when the carrier clutch solenoid is energized, and the carrier clutch is disengaged when the carrier clutch solenoid is de-energized.

12. The knee prosthesis or orthosis (10) of claim 11, where the ring clutch (400, 500, 600) is a unidirectional clutch, such that in the engaged state, the ring clutch (400, 500, 600) allows unidirectional rotation of the common ring gear (52, 412, 512) relative to ground, where the unidirectional ring clutch (400, 500, 600) preferably is a capstan clutch.

13. The knee prosthesis or orthosis (10) of claim 11, wherein the ring clutch (400, 500, 600) is selectively unidirectional or bidirectional, wherein preferably engagement of the first and second ring clutches (400, 500, 600) prevents or inhibits rotation of the common ring gear (52, 412, 512) in both directions, and the selective disengagement of one of the first and second ring clutches (400, 500, 600) allows unidirectional rotation of the common ring gear (52, 412, 512) relative to ground in one of two directions, and the selective disengagement of both the first and second ring clutches (400, 500, 600) allows bidirectional rotation of the common ring gear (52, 412, 512) relative to ground.

14. The knee prosthesis or orthosis (10) of claim 7, wherein the ring clutch (400, 500, 600) is a bidirectional clutch, such that in the engaged state, the ring clutch (400, 500, 600) prevents or inhibits rotation of the common ring gear (52, 412, 512) relative to ground in both directions.

15. The knee prosthesis or orthosis (10) of claim 7, wherein the prosthesis or orthosis (10) is configured to be in a first state (210), a second state (220) or a third state(230), wherein the first state (210) includes the ring clutch (400, 500, 600) being engaged and the carrier clutch being disengaged, wherein the second state (220) includes the ring clutch (400, 500, 600) being disengaged and the carrier clutch being engaged, and wherein the third state (230) includes the ring clutch (400, 500, 600) being disengaged and the carrier clutch being disengaged, wherein preferably the prosthesis or orthosis (10) further comprises a device control unit (120), the device control unit (120) configures: (1) the prosthesis or orthosis (10) into the first state (210) during early and middle stance phase; (2) the prosthesis or orthosis (10) into the second state (220) in late stance phase and during the flexion portion of swing phase; and (3) the prosthesis or orthosis (10) into the third state (230) during the extension portion of swing phase.

## Patentansprüche

1. Knieprothese oder -Orthese (10), umfassend:
mindestens ein bewegliches Gelenk (12) und
eine Drehmomentmodulationseinheit (14, 300), wobei die Drehmomentmodulationseinheit (14, 300) dazu ausgestaltet ist, dem mindestens einen beweglichen Gelenk (12) ein steuerbares Drehmoment aufzuerlegen, wobei die Drehmomentmodulationseinheit (14, 300) einen Motor und/oder eine Bremse (302) aufweist, wobei der Motor und/oder die Bremse (302) über ein elektronisch gesteuertes Getriebe an das mindestens eine bewegliche Gelenk (12) gekoppelt ist, **dadurch gekennzeichnet, dass** das elektronisch gesteuerte Getriebe ein Zweiganggetriebe ist.

2. Knieprothese oder -Orthese (10) nach Anspruch 1, wobei das Zweiganggetriebe eine hohe Getriebeübersetzung und eine niedrige Getriebeübersetzung aufweist, wobei das Zweiganggetriebe dazu ausgestaltet ist, innerhalb einer Schreitperiode während eines Gangzyklus zwischen der hohen Getriebeübersetzung und der niedrigen Getriebeübersetzung zu schalten, wobei der Gangzyklus eine Standphase und eine Schwungphase aufweist,
wobei die hohe Getriebeübersetzung eine Verhaltenscharakteristik mit hohem Drehmoment, niedriger Drehzahl und hoher Impedanz bereitstellt, die zu der Standphase des Gangzyklus konfiguriert ist, und wobei die hohe Getriebeübersetzung dazu ausgestaltet ist, ein Gewicht der Prothese oder Orthese (10) eines Benutzers zu stützen, wenn er auf der Knieprothese oder -Orthese (10) steht,
wobei die niedrige Getriebeübersetzung eine Verhaltenscharakteristik mit hoher Drehzahl, niedrigem Drehmoment und niedriger Impedanz bereitstellt, die für die Schwungphase des Gangzyklus geeignet ist, und wobei die niedrige Getriebeübersetzung so ausgestaltet ist, dass die Prothese oder Orthese (10) einem Kniegelenk freies Schwingen gestatten kann, wenn die Knieprothese oder -Orthese (10) das Gewicht des Benutzers nicht trägt.

3. Knieprothese oder -Orthese (10) nach Anspruch 2, wobei sich die hohe und die niedrige Getriebeübersetzung um mindestens etwa eine Größenordnung unterscheiden.

4. Knieprothese oder -Orthese (10) nach Anspruch 2,
- wobei die hohe Getriebeübersetzung dazu ausgestaltet ist, ein unidirektionales Verhalten aufzuweisen, wobei das Zweiganggetriebe dazu ausgestaltet ist, ein Drehmoment gegen Knieflexion zu erzeugen, aber bei Widerstand gegen eine Knie-Extension rutscht, und/oder
- wobei die niedrige Getriebeübersetzung dazu ausgestaltet ist, ein asymmetrisches Verhalten aufzuweisen, wobei das Zweiganggetriebe dazu ausgestaltet ist, Extension mit einem niedrigen Widerstand zu widerstehen, Flexion jedoch mit einem hohen Widerstand zu widerstehen.

5. Knieprothese oder-Orthese (10) nach Anspruch 2, wobei das Zweiganggetriebe dazu ausgestaltet ist, sich in mindestens einem ersten Zustand (210), einem zweiten Zustand (220) oder einem dritten Zustand (230) zu befinden, wobei das Zweiganggetriebe im ersten Zustand (210) die hohe Getriebeübersetzung bereitstellt, wenn es Flexion widersteht oder Extension bereitstellt, während es bei Widerstand gegen Extension oder Bereitstellung von Flexion wenig bis kein Drehmoment bereitstellt, das Zweiganggetriebe im zweiten Zustand (220) die niedrige Getriebeübersetzung bereitstellt, wobei die niedrige Getriebeübersetzung bidirektional ist, und das Zweiganggetriebe im dritten Zustand (230) die niedrige Getriebeübersetzung bereitstellt, wenn es Extension widersteht oder Flexion bereitstellt, während es auch hohen Widerstand bereitstellt, wenn es Flexion widersteht oder Extension bereitstellt, wobei vorzugsweise das Zweiganggetriebe ferner für einen vierten Zustand ausgestaltet ist, wobei das Zweiganggetriebe im vierten Zustand die hohe Getriebeübersetzung bereitstellt, wobei die hohe Getriebeübersetzung bidirektional ist.

6. Knieprothese oder -Orthese (10) nach Anspruch 2, wobei das Zweiganggetriebe dazu ausgestaltet ist, sich in mindestens einem neutralen Zustand zu befinden, in dem das Zweiganggetriebe von dem Motor und/oder der Bremse (302) ausgerückt ist.

7. Knieprothese oder -Orthese (10) nach Anspruch 2, wobei das Zweiganggetriebe ein Planetengetriebe (310) aufweist, wobei das Planetengetriebe mindestens eine Planeten-Eingangsstufe und eine Planeten-Ausgangsstufe aufweist, wobei die Planeten-Eingangsstufe mindestens ein Eingangssonnenrad (60) und ein Eingangsträgerverbindungsglied (64, 164, 165) aufweist, wobei die Planeten-Ausgangsstufe ein Ausgangsträgerverbindungsglied (68, 168) aufweist,
wobei der Motor und/oder die Bremse (302) an das Eingangssonnenrad (60) gekoppelt sind und mindestens ein bewegliches Gelenk (12) direkt oder indirekt an das Ausgangsträgerverbindungsglied (68, 168) gekoppelt ist,
wobei das Planetengetriebe ferner ein gemeinsames Hohlrad (52, 412, 512) aufweist,
wobei das Zweiganggetriebe mindestens eine Ringkupplung (400, 500, 600) und eine Trägerkupplung aufweist, wobei die Ringkupplung (400, 500, 600) so konfigurierbar ist, dass sie sich in einem eingerückten Zustand oder einem ausgerückten Zustand befindet, wobei die Trägerkupplung so konfigurierbar ist, dass sie sich in einem eingerückten Zustand oder einem ausgerückten Zustand befindet,
wobei die Ringkupplung (400, 500, 600) im ausgerückten Zustand dem gemeinsamen Hohlrad (52, 412, 512) gestattet, sich relativ zu Masse zu drehen, und wobei die Ringkupplung (400, 500, 600) im eingerückten Zustand verhindert, dass sich das gemeinsame Zahnrad relativ zu Masse dreht, oder dieses hemmt,
wobei die Trägerkupplung im ausgerückten Zustand gestattet, dass sich das Eingangsträgerverbindungsglied (64, 164, 165) relativ zu Masse dreht, und wobei die Trägerkupplung im eingerückten Zustand verhindert, dass sich das Eingangsträgerverbindungsglied (64, 164, 165) relativ zu Masse dreht, oder dieses hemmt.

8. Knieprothese oder -Orthese (10) nach Anspruch 7, wobei die Planeten-Eingangsstufe ferner mindestens einen Satz von drei Eingangsplanetenrädern (56) aufweist und die Planeten-Ausgangsstufe ferner ein Ausgangssonnenrad (62) und mindestens einen Satz von drei Ausgangsplanetenrädern (58) aufweist.

9. Knieprothese oder -Orthese (10) nach Anspruch 7, wobei die Ringkupplung (400, 500, 600) und die Trägerkupplung elektromagnetisch betätigte Kupplungen sind.

10. Knieprothese oder -Orthese (10) nach Anspruch 8, wobei das Eingangsträgerverbindungsglied (64, 164, 165) dazu ausgestaltet ist, entlang einer Eingangswelle zu translatieren, und wobei bei Strombeaufschlagung eines an Masse befestigten Solenoids das aus dem Solenoid resultierende Magnetfeld das Eingangsträgerverbindungsglied (64, 164, 165) entlang der Eingangswelle zieht, um einen Kontakt mit Masse herzustellen.

11. Knieprothese oder -Orthese (10) nach Anspruch 9, wobei die Ringkupplung (400, 500, 600) eingerückt ist, wenn das Ringkupplungssolenoid (334) stromlos ist, und die Ringkupplung (400, 500, 600) ausgerückt ist, wenn das Ringkupplungssolenoid (334) mit Strom beaufschlagt ist, und wobei die Trägerkupplung eingerückt ist, wenn das Trägerkupplungssolenoid mit Strom beaufschlagt ist, und die Trägerkupplung ausgerückt ist, wenn das Trägerkupplungssolenoid stromlos ist.

12. Knieprothese oder -Orthese (10) nach Anspruch 11, wobei die Ringkupplung (400, 500, 600) eine unidirektionale Kupplung ist, so dass die Ringkupplung (400, 500, 600) im eingerückten Zustand eine unidirektionale Drehung des gemeinsamen Hohlrads (52, 412, 512) relativ zu Masse gestattet, wobei die unidirektionale Ringkupplung (400, 500, 600) vorzugsweise eine Seilkupplung ist.

13. Knieprothese oder -Orthese (10) nach Anspruch 11, wobei die Ringkupplung (400, 500, 600) selektiv unidirektional oder bidirektional ist, wobei vorzugsweise ein Einrücken der ersten und der zweiten Ringkupplung (400, 500, 600) eine Drehung des gemeinsamen Hohlrads (52, 412, 512) in beiden Richtungen verhindert oder hemmt, und das selektive Ausrücken der ersten oder der zweiten Ringkupplung (400, 500, 600) eine unidirektionale Drehung des gemeinsamen Hohlrads (52, 412, 512) relativ zu Masse in einer von zwei Richtungen gestattet, und das selektive Ausrücken sowohl der ersten als auch der zweiten Ringkupplung (400, 500, 600) eine bidirektionale Drehung des gemeinsamen Hohlrads (52, 412, 512) relativ zu Masse gestattet.

14. Knieprothese oder -Orthese (10) nach Anspruch 7, wobei die Ringkupplung (400, 500, 600) eine bidirektionale Kupplung ist, so dass die Ringkupplung (400, 500, 600) im eingerückten Zustand eine Drehung des gemeinsamen Hohlrads (52, 412, 512) relativ zu Masse in beiden Richtungen verhindert oder hemmt.

15. Knieprothese oder -Orthese (10) nach Anspruch 7, wobei die Prothese oder Orthese (10) dazu ausgestaltet ist, sich in einem ersten Zustand (210), einem zweiten Zustand (220) oder einem dritten Zustand (230) zu befinden, wobei im ersten Zustand (210) die Ringkupplung (400, 500, 600) eingerückt und die Trägerkupplung ausgerückt ist, wobei im zweiten Zustand (220) die Ringkupplung (400, 500, 600) ausgerückt und die Trägerkupplung eingerückt ist und wobei im dritten Zustand (230) die Ringkupplung (400, 500, 600) ausgerückt und die Trägerkupplung ausgerückt ist, wobei vorzugsweise die Prothese oder Orthese (10) ferner eine Vorrichtungssteuereinheit (120) umfasst, wobei die Vorrichtungssteuereinheit (120): (1) die Prothese oder Orthese (10) während der frühen und mittleren Standphase in den ersten Zustand (210) konfiguriert, (2) die Prothese oder Orthese (10) in der späten Standphase und während des Flexionsteils der Schwungphase in den zweiten Zustand (220) konfiguriert, und (3) die Prothese oder Orthese (10) während des Extensionsteils der Schwungphase in den dritten Zustand (230) konfiguriert.

## Revendications

1. Prothèse ou orthèse du genou (10), comprenant :
au moins une articulation mobile (12) ; et
une unité de modulation de couple (14, 300), l'unité de modulation de couple (14, 300) étant configurée pour imposer un couple réglable à l'au moins une articulation mobile (12), l'unité de modulation de couple (14, 300) comprenant au moins l'un parmi un moteur et un frein (302), l'au moins un parmi le moteur et le frein (302) étant couplé à l'au moins une articulation mobile (12) par l'intermédiaire d'une transmission à commande électronique, **caractérisée en ce que** la transmission à commande électronique est une transmission à deux vitesses.

2. Prothèse ou orthèse du genou (10) de la revendication 1, dans laquelle la transmission à deux vitesses comprend un rapport de transmission à grande vitesse et un rapport de transmission à faible vitesse, la transmission à deux vitesses étant configurée pour commuter entre le rapport de transmission à grande vitesse et le rapport de transmission à faible vitesse pendant une période intra-foulée lors d'un cycle de marche, le cycle de marche comprenant une phase d'appui et une phase d'oscillation,
dans laquelle le rapport de transmission à grande vitesse fournit une caractéristique de comportement à couple élevé, faible vitesse et impédance élevée configurée pour la phase d'appui du cycle de marche, et dans laquelle le rapport de transmission à grande vitesse est configuré pour supporter un poids de la prothèse ou de l'orthèse (10) d'un utilisateur lorsqu'il est debout sur la prothèse ou l'orthèse du genou (10),
dans laquelle le rapport de transmission à faible vitesse fournit une caractéristique de comportement à grande vitesse, faible couple et faible impédance adaptée à la phase d'oscillation du cycle de marche, et dans laquelle le rapport de transmission à faible vitesse est configuré pour permettre à la prothèse ou à l'orthèse (10) de laisser l'articulation du genou osciller librement lorsque la prothèse ou l'orthèse du genou (10) ne supporte pas le poids de l'utilisateur.

3. Prothèse ou orthèse du genou (10) de la revendication 2, dans laquelle les rapports de transmission à grande vitesse et à faible vitesse sont différents d'au moins environ un ordre de grandeur.

4. Prothèse ou orthèse du genou (10) de la revendication 2,
- dans laquelle le rapport de transmission à grande vitesse est configuré pour avoir un comportement unidirectionnel, dans laquelle la transmission à deux vitesses est configurée pour générer un couple contre la flexion du genou, mais glisse lorsqu'elle résiste à l'extension du genou, et/ou
- dans laquelle le rapport de transmission à faible vitesse est configuré pour avoir un comportement asymétrique, dans laquelle la transmission à deux vitesses est configurée pour résister à l'extension avec une faible résistance tout en résistant à la flexion avec une résistance élevée.

5. Prothèse ou orthèse du genou (10) de la revendication 2, dans laquelle la transmission à deux vitesses est configurée pour être dans au moins un premier état (210), un deuxième état (220) ou un troisième état (230), la transmission à deux vitesses dans le premier état (210) fournit le rapport de transmission à grande vitesse lorsqu'elle résiste à la flexion ou fournit une extension, tout en fournissant peu ou pas de couple lorsqu'elle résiste à l'extension ou fournit une flexion, la transmission à deux vitesses dans le deuxième état (220) fournit le rapport de transmission à faible vitesse, le rapport de transmission à faible vitesse étant bidirectionnel ; et la transmission à deux vitesses dans le troisième état (230) fournit le rapport de transmission à faible vitesse lorsqu'elle résiste à l'extension ou fournit une flexion, tout en fournissant également une résistance élevée lorsqu'elle résiste à la flexion ou fournit une extension, dans laquelle de préférence la transmission à deux vitesses est en outre configurée pour un quatrième état, la transmission à deux vitesses dans le quatrième état fournissant le rapport de transmission à grande vitesse, le rapport de transmission à grande vitesse étant bidirectionnel.

6. Prothèse ou orthèse du genou (10) de la revendication 2, dans laquelle la transmission à deux vitesses est configurée pour être dans au moins un état neutre, dans lequel la transmission à deux vitesses est désengagée d'au moins l'un parmi le moteur et le frein (302).

7. Prothèse ou orthèse du genou (10) de la revendication 2, dans laquelle la transmission à deux vitesses comprend une transmission à engrenages planétaires (310), la transmission à engrenages planétaires comprenant au moins un étage planétaire d'entrée et un étage planétaire de sortie, l'étage planétaire d'entrée comprenant au moins un planétaire d'entrée (60) et une liaison de support d'entrée (64, 164, 165), l'étage planétaire de sortie comprenant une liaison de support de sortie (68, 168),
dans laquelle l'au moins un parmi un moteur et un frein (302) est couplé au planétaire d'entrée (60) et au moins une articulation mobile (12) est couplée directement ou indirectement à la liaison de support de sortie (68, 168),
dans laquelle la transmission planétaire comprend en outre une couronne commune (52, 412, 512),
dans laquelle la transmission à deux vitesses comprend au moins un embrayage annulaire (400, 500, 600) et un embrayage de support, l'embrayage annulaire (400, 500, 600) pouvant être configuré pour être dans un état engagé ou un état désengagé, l'embrayage de support pouvant être configuré pour être dans un état engagé ou un état désengagé,
dans laquelle, dans l'état désengagé, l'embrayage annulaire (400, 500, 600) permet à la couronne commune (52, 412, 512) de tourner par rapport au sol, et dans laquelle dans l'état engagé, l'embrayage annulaire (400, 500, 600) empêche ou inhibe la rotation de la couronne commune par rapport au sol.
dans laquelle, dans l'état désengagé, l'embrayage de support permet à la liaison de support d'entrée (64, 164, 165) de tourner par rapport au sol, et dans laquelle dans l'état engagé, l'embrayage de support empêche ou inhibe la rotation de la liaison de support d'entrée (64, 164, 165) par rapport au sol.

8. Prothèse ou orthèse du genou (10) de la revendication 7, dans laquelle l'étage planétaire d'entrée comprend en outre au moins un ensemble de trois satellites d'entrée (56), et l'étage planétaire de sortie comprend en outre un planétaire de sortie (62) et au moins un ensemble de trois satellites de sortie (58).

9. Prothèse ou orthèse du genou (10) de la revendication 7, dans laquelle l'embrayage annulaire (400, 500, 600) et l'embrayage de support sont des embrayages à commande par solénoïde.

10. Prothèse ou orthèse du genou (10) de la revendication 8, dans laquelle la liaison de support d'entrée (64, 164, 165) est configurée pour se déplacer en translation le long d'un arbre d'entrée, et dans laquelle, lors de l'alimentation d'un solénoïde fixé au sol, le champ magnétique résultant du solénoïde tire la liaison de support d'entrée (64, 164, 165) le long de l'arbre d'entrée pour entrer en contact avec le sol.

11. Prothèse ou orthèse du genou (10) de la revendication 9, dans laquelle l'embrayage annulaire (400, 500, 600) est engagé lorsque le solénoïde (334) d'embrayage annulaire est mis hors tension, et l'embrayage annulaire (400, 500, 600) est désengagé lorsque le solénoïde (334) d'embrayage annulaire est alimenté, et dans laquelle l'embrayage de support est engagé lorsque le solénoïde d'embrayage de support est alimenté, et l'embrayage de support est désengagé lorsque le solénoïde d'embrayage de support est mis hors tension.

12. Prothèse ou orthèse du genou (10) de la revendication 11, dans laquelle l'embrayage annulaire (400, 500, 600) est un embrayage unidirectionnel, de sorte que dans l'état engagé, l'embrayage annulaire (400, 500, 600) permet une rotation unidirectionnelle de la couronne commune (52, 412, 512) par rapport au sol, où l'embrayage annulaire unidirectionnel (400, 500, 600) est de préférence un embrayage au cabestan.

13. Prothèse ou orthèse du genou (10) de la revendication 11, dans laquelle l'embrayage annulaire (400, 500, 600) est sélectivement unidirectionnel ou bidirectionnel, dans laquelle de préférence l'engagement des premier et second embrayages annulaires (400, 500, 600) empêche ou inhibe la rotation de la couronne commune (52, 412, 512) dans les deux sens, et le désengagement sélectif de l'un des premier et second embrayages annulaires (400, 500, 600) permet une rotation unidirectionnelle de la couronne commune (52, 412, 512) par rapport au sol dans l'un des deux sens, et le désengagement sélectif des premier et second embrayages annulaires (400, 500, 600) tous deux permet une rotation bidirectionnelle de la couronne commune (52, 412, 512) par rapport au sol.

14. Prothèse ou orthèse du genou (10) de la revendication 7, dans laquelle l'embrayage annulaire (400, 500, 600) est un embrayage bidirectionnel, de sorte que dans l'état engagé, l'embrayage annulaire (400, 500, 600) empêche ou inhibe la rotation de la couronne commune (52, 412, 512) par rapport au sol dans les deux sens.

15. Prothèse ou orthèse du genou (10) de la revendication 7, dans laquelle la prothèse ou l'orthèse (10) est configurée pour être dans un premier état (210), un deuxième état (220) ou un troisième état (230), dans laquelle le premier état (210) comprend l'embrayage annulaire (400, 500, 600) engagé et l'embrayage de support désengagé, dans laquelle le deuxième état (220) comprend l'embrayage annulaire (400, 500, 600) désengagé et l'embrayage de support engagé, et dans laquelle le troisième état (230) comprend l'embrayage annulaire (400, 500, 600) désengagé et l'embrayage de support désengagé, dans laquelle de préférence la prothèse ou l'orthèse (10) comprend en outre une unité de commande de dispositif (120), l'unité de commande de dispositif (120) configure : (1) la prothèse ou l'orthèse (10) dans le premier état (210) en phase d'appui précoce et intermédiaire ; (2) la prothèse ou l'orthèse (10) dans le deuxième état (220) en phase d'appui tardif et pendant la partie de flexion de la phase d'oscillation ; et (3) la prothèse ou l'orthèse (10) dans le troisième état (230) pendant la partie d'extension de la phase d'oscillation.
